# EUROPEAN PATENT APPLICATION

(11) **EP 4 102 221 A1**
(43) Date of publication of application: **14.12.2022**
(21) Application number: 22177797.2
(22) Date of filing: 08.06.2022
(51) Int. Cl.: G01N 30/24, B01D 15/14, G01N 30/86, G01N 35/00

(54) **AUTO OUTLIER INJECTION IDENTIFICATION**

(30) Priority: 10.06.2021 US 202117344773
(71) Applicant: Thermo Finnigan LLC, San Jose, CA 95134 (US)
(72) Inventor: ZHENG, Xin, Austin, 78728 (US)
(74) Representative: Boult Wade Tennant LLP

(57) **Abstract**

A method for scientific instrument support includes obtaining a chromatographic data. The method includes one of (a) calculating an intensity score for the chromatographic data and identifying an injection miss when the intensity score is below a score threshold or (b) applying a machine learning model to classify the chromatographic data. The method further includes notifying a user of an injection miss or injection error.

## Description

### FIELD

The present disclosure generally relates to the field of chromatography including identification of outlier injections.

### INTRODUCTION

Chromatography is a useful technique for analyzing compounds of a complex sample. A chromatography column having a retention medium or stationary phase can be used to separate the compounds based on their affinity to the retention medium. The time it takes a compound to traverse the column (retention time) is compound dependent as compounds with higher affinity for the stationary phase can be retained in the column for a longer period of time than compounds that have fewer interactions with the stationary phase.

High throughput chromatography systems utilize autosamplers and can be operated without human intervention for multiple hours. Chromatography systems including autosamplers may include a complex arrangement of movable components, sensors, input and output ports, energy sources, and consumable components. Failures or changes in any part of this arrangement may result in a "downed" instrument, one that is not able to perform its intended function.

### SUMMARY

In a first aspect, a method for scientific instrument support can include obtaining a chromatographic data; calculating an intensity score for the chromatographic data; identifying an injection miss when the intensity score is below a score threshold; and notifying a user of an injection miss.

In various embodiments of the first aspect, the intensity score can include a total signal intensity, an average signal intensity, average area of a number of peaks, a number of peaks above an intensity threshold, an area above the intensity threshold, or a chromatographic similarity score.

In various embodiments of the first aspect, the method can further include pausing or stopping a chromatography system until the cause of the injection miss is corrected or a user input to resume or restart is received.

In a second aspect, a scientific instrument support apparatus can include first logic to obtain a chromatographic data; second logic to calculate an intensity score for the chromatographic data and identify an injection miss when the intensity score is below a threshold; and third logic to notify a user of an injection miss.

In various embodiments of the second aspect, the intensity score can include a total signal intensity, an average signal intensity, average area of a number of peaks, a number of peaks above an intensity threshold, an area above the intensity threshold, or a chromatographic similarity score.

In various embodiments of the second aspect, the third logic can be further configured to pause or stop a chromatography system until the cause of the injection miss is corrected or a user input to resume or restart is received.

In various embodiments of the second aspect, the first logic, the second logic, and the third logic can be implemented by a common computing device.

In various embodiments of the second aspect, at least one of the first logic, the second logic, and the third logic can be implemented by a computing device remote from the scientific instrument.

In various embodiments of the second aspect, at least one of the first logic, the second logic, and the third logic can be implemented by a user computing device.

In various embodiments of the second aspect, at least one of the first logic, the second logic, and the third logic can be implemented in the scientific instrument.

In a third aspect, a method for scientific instrument support can include obtaining a chromatographic data; applying a machine learning model to classify the chromatographic data; and notifying a user when the machine learning model classifies the chromatographic data as a chromatographic error. The invention also extends to a computer program containing program code which, when executed, carries out the method steps of the third aspect.

In various embodiments of the third aspect, the method can further include providing a recommendation to the user to correct the cause of the chromatographic error.

In various embodiments of the third aspect, the method can further include pausing or stopping a chromatography system until the cause of the chromatographic error is corrected or a user input to resume or restart is received.

In a fourth aspect, a scientific instrument support apparatus can include first logic to obtain a chromatographic data; second logic to apply a machine learning model to classify the chromatographic data; and third logic to notify a user when the machine learning model classifies the chromatographic data as a chromatographic error.

In various embodiments of the fourth aspect, the third logic can be further configured to provide a recommendation to the user to correct the cause of the chromatographic error.

In various embodiments of the fourth aspect, the third logic can be further configured to pause or stop a chromatography system until the cause of the injection miss is corrected or a user input to resume or restart is received.

In various embodiments of the fourth aspect, the first logic, the second logic, and the third logic can be implemented by a common computing device.

In various embodiments of the fourth aspect, at least one of the first logic, the second logic, and the third logic can be implemented by a computing device remote from the scientific instrument.

In various embodiments of the fourth aspect, at least one of the first logic, the second logic, and the third logic can be implemented by a user computing device.

In various embodiments of the fourth aspect, at least one of the first logic, the second logic, and the third logic can be implemented in the scientific instrument.

### DRAWINGS

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.
FIG. 1 is a block diagram of an example scientific instrument support module for performing support operations, in accordance with various embodiments.
FIG. 2 is a flow diagram of an example method of performing support operations, in accordance with various embodiments.
FIG. 3 is an example of a graphical user interface that may be used in the performance of some or all of the support methods disclosed herein, in accordance with various embodiments.
FIG. 4 is a block diagram of an example computing device that may perform some or all of the scientific instrument support methods disclosed herein, in accordance with various embodiments.
FIG. 5 is a block diagram of an example scientific instrument support system in which some or all of the scientific instrument support methods disclosed herein may be performed, in accordance with various embodiments.
Figure 6 is a diagram of an exemplary chromatography system.
Figures 7A-7F are illustrations of a plurality of chromatograms, in accordance with various embodiments.
Figure 8 is a flow diagram illustrating an exemplary method of identifying missed injections, in accordance with various embodiments.
Figure 9 is a flow diagram illustrating an exemplary method of classifying chromatograms using a machine learning model, in accordance with various embodiments.

It is to be understood that the figures are not necessarily drawn to scale, nor are the objects in the figures necessarily drawn to scale in relationship to one another.
The figures are depictions that are intended to bring clarity and understanding to various embodiments of apparatuses, systems, and methods disclosed herein. Wherever possible, the same reference numbers will be used throughout the drawings to refer to the same or like parts. Moreover, it should be appreciated that the drawings are not intended to limit the scope of the present teachings in any way.

### DESCRIPTION OF VARIOUS EMBODIMENTS

The section headings used herein are for organizational purposes only and are not to be construed as limiting the described subject matter in any way.

Disclosed herein are scientific instrument support systems, as well as related methods, computing devices, and computer-readable media. For example, in some embodiments, a method for scientific instrument support can include obtaining a chromatographic data; calculating an intensity score for the chromatographic data; identifying an injection miss when the intensity score is below a score threshold; notifying a user of an injection miss.

The scientific instrument support embodiments disclosed herein may achieve improved performance relative to conventional approaches. For example, a sampling error, such as a bent of clogged needle, that goes unnoticed by the user while the instrument operates unattended, can result in many unusable chromatographic data sets. Detection of such sampling errors in near real time can provide the user an opportunity to correct the issue and restart or resume collecting data, significantly reducing the lost time and resources that would have otherwise been used collecting unusable data.

In the following detailed description, reference is made to the accompanying drawings that form a part hereof wherein like numerals designate like parts throughout, and in which is shown, by way of illustration, embodiments that may be practiced. It is to be understood that other embodiments may be utilized, and structural or logical changes may be made, without departing from the scope of the present disclosure. Therefore, the following detailed description is not to be taken in a limiting sense.

Various operations may be described as multiple discrete actions or operations in turn, in a manner that is most helpful in understanding the subject matter disclosed herein. However, the order of description should not be construed as to imply that these operations are necessarily order dependent. In particular, these operations may not be performed in the order of presentation. Operations described may be performed in a different order from the described embodiment. Various additional operations may be performed, and/or described operations may be omitted in additional embodiments.

For the purposes of the present disclosure, the phrases "A and/or B" and "A or B" mean (A), (B), or (A and B). For the purposes of the present disclosure, the phrases "A, B, and/or C" and "A, B, or C" mean (A), (B), (C), (A and B), (A and C), (B and C), or (A, B, and C). Although some elements may be referred to in the singular (e.g., "a processing device"), any appropriate elements may be represented by multiple instances of that element, and vice versa. For example, a set of operations described as performed by a processing device may be implemented with different ones of the operations performed by different processing devices.

The description uses the phrases "an embodiment," "various embodiments," and "some embodiments," each of which may refer to one or more of the same or different embodiments. Furthermore, the terms "comprising," "including," "having," and the like, as used with respect to embodiments of the present disclosure, are synonymous. When used to describe a range of dimensions, the phrase "between X and Y" represents a range that includes X and Y. As used herein, an "apparatus" may refer to any individual device or collection of devices. The drawings are not necessarily to scale.

All literature and similar materials cited in this application, including but not limited to, patents, patent applications, articles, books, treatises, and internet web pages are expressly incorporated by reference in their entirety for any purpose. Unless described otherwise, all technical and scientific terms used herein have a meaning as is commonly understood by one of ordinary skill in the art to which the various embodiments described herein belongs.

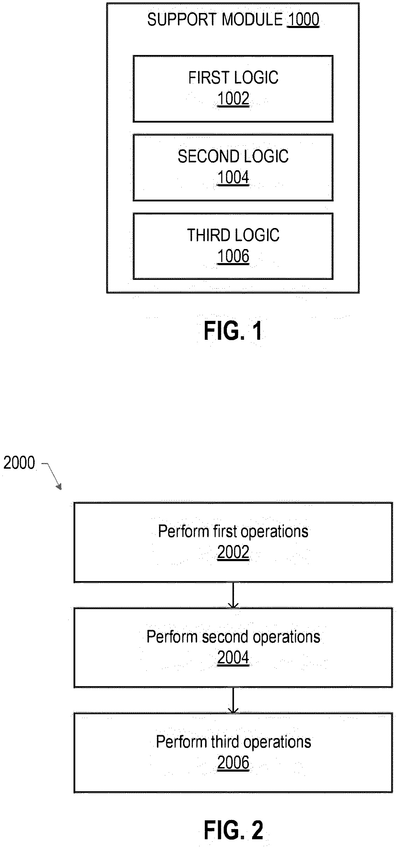

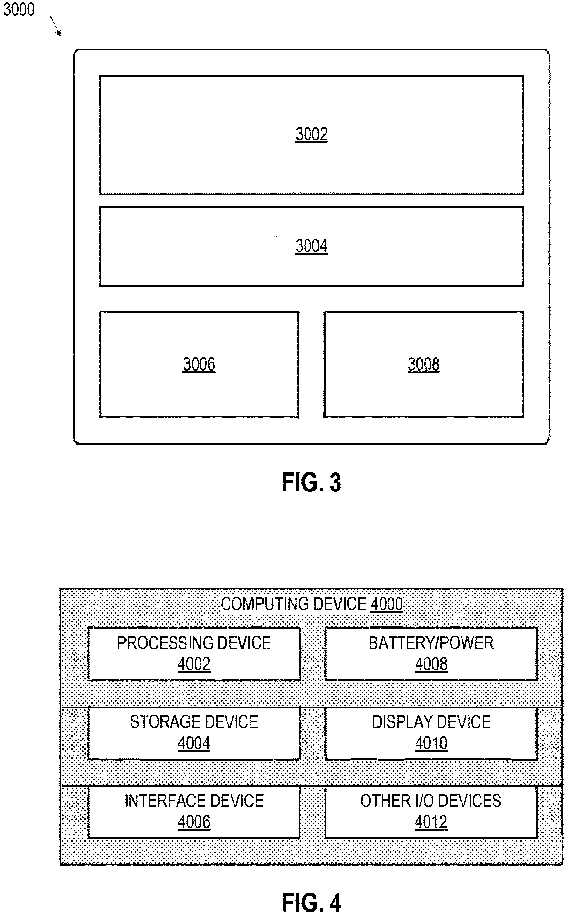

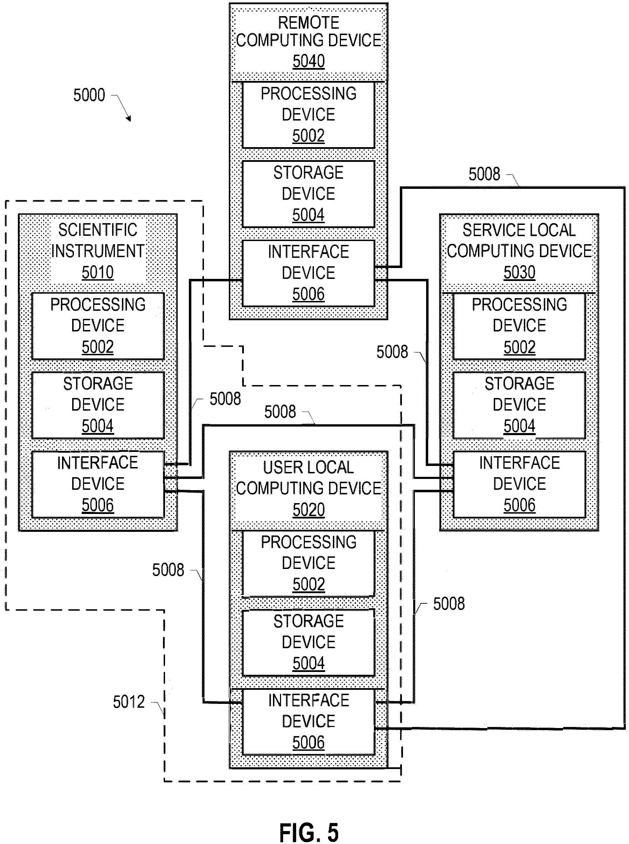

FIG. 1 is a block diagram of a scientific instrument support module 1000 for performing support operations, in accordance with various embodiments. The scientific instrument support module 1000 may be implemented by circuitry (e.g., including electrical and/or optical components), such as a programmed computing device. The logic of the scientific instrument support module 1000 may be included in a single computing device or may be distributed across multiple computing devices that are in communication with each other as appropriate. Examples of computing devices that may, singly or in combination, implement the scientific instrument support module 1000 are discussed herein with reference to the computing device 4000 of FIG. 4, and examples of systems of interconnected computing devices, in which the scientific instrument support module 1000 may be implemented across one or more of the computing devices, is discussed herein with reference to the scientific instrument support system 5000 of FIG. 5.

The scientific instrument support module 1000 may include first logic 1002, second logic 1004, and third logic 1006. As used herein, the term "logic" may include an apparatus that is to perform a set of operations associated with the logic. For example, any of the logic elements included in the support module 1000 may be implemented by one or more computing devices programmed with instructions to cause one or more processing devices of the computing devices to perform the associated set of operations. In a particular embodiment, a logic element may include one or more non-transitory computer-readable media having instructions thereon that, when executed by one or more processing devices of one or more computing devices, cause the one or more computing devices to perform the associated set of operations. As used herein, the term "module" may refer to a collection of one or more logic elements that, together, perform a function associated with the module. Different ones of the logic elements in a module may take the same form or may take different forms. For example, some logic in a module may be implemented by a programmed general-purpose processing device, while other logic in a module may be implemented by an application-specific integrated circuit (ASIC). In another example, different ones of the logic elements in a module may be associated with different sets of instructions executed by one or more processing devices.

The first logic 1002 may obtain a chromatogram. In various embodiments, the first logic 1002 can instruct the scientific instrument to process a sample, such as by using an autosampler to take an aliquot from a sample vial, inject the aliquot onto a chromatography column, elute the components of the sample from the column, and use an analyzer to collect data on the components of the sample. First logic 1002 can further receive the data from the analyzer and store the data into a database, filesystem, or the like for further processing.

The second logic 1004 may take the data received from the scientific instrument by first logic 1002 and determine if the chromatogram is representative of a successful result or if a problem such as a missed injection occurred. In various embodiments, second logic 1004 can include calculating an intensity score for the chromatogram and comparing the score to a threshold. Alternatively, second logic 1004 can apply a machine learning model to the chromatogram to classify the chromatogram as successful or abnormal.

The third logic 1006 may notify a user of a problem with a chromatogram, as determined by second logic 1004. In various embodiments, third logic 1006 can provide recommendations to the user to correct the problem. Additionally, third logic 1006 may allow for preprogrammed responses such as pausing the scientific instrument until a user performs a corrective action or acknowledges the notification and resumes the experiment.

FIG. 2 is a flow diagram of a method 2000 of performing support operations, in accordance with various embodiments. Although the operations of the method 2000 may be illustrated with reference to particular embodiments disclosed herein (e.g., the scientific instrument support modules 1000 discussed herein with reference to FIG. 1, the GUI 3000 discussed herein with reference to FIG. 3, the computing devices 4000 discussed herein with reference to FIG. 4, and/or the scientific instrument support system 5000 discussed herein with reference to FIG. 5), the method 2000 may be used in any suitable setting to perform any suitable support operations. Operations are illustrated once each and in a particular order in FIG. 2, but the operations may be reordered and/or repeated as desired and appropriate (e.g., different operations performed may be performed in parallel, as suitable).

At 2002, first operations may be performed. For example, the first logic 1002 of a support module 1000 may perform the operations of 2002. The first operations may include instructing the scientific instrument to process a sample, such as by using an autosampler to take an aliquot from a sample vial, injecting the aliquot onto a chromatography column, eluting the components of the sample from the column, and using an analyzer to collect data on the components of the sample. First operations can also include receiving data from the analyzer and store the data into a database, filesystem, or the like for further processing.

At 2004, second operations may be performed. For example, the second logic 1004 of a support module 1000 may perform the operations of 2004. The second operations may include determining if the chromatogram is representative of a successful result or if a problem such as a missed injection occurred. In various embodiments, the second operations can include calculating an intensity score for the chromatogram and comparing the score to a threshold. Alternatively, second operations can include applying a machine learning model to the chromatogram to classify the chromatogram as successful or abnormal.

At 2006, third operations may be performed. For example, the third logic 1006 of a support module 1000 may perform the operations of 2006. The third operations may include notifying a user of a problem with a chromatogram, as determined by second operations. In various embodiments, third operations can include providing recommendations to the user to correct the problem. Additionally, third operations may allow for preprogrammed responses such as pausing the scientific instrument until a user performs a corrective action or acknowledges the notification and resumes the experiment.

The scientific instrument support methods disclosed herein may include interactions with a human user (e.g., via the user local computing device 5020 discussed herein with reference to FIG. 5). These interactions may include providing information to the user (e.g., information regarding the operation of a scientific instrument such as the scientific instrument 5010 of FIG. 5, information regarding a sample being analyzed or other test or measurement performed by a scientific instrument, information retrieved from a local or remote database, or other information) or providing an option for a user to input commands (e.g., to control the operation of a scientific instrument such as the scientific instrument 5010 of FIG. 5, or to control the analysis of data generated by a scientific instrument), queries (e.g., to a local or remote database), or other information. In some embodiments, these interactions may be performed through a graphical user interface (GUI) that includes a visual display on a display device (e.g., the display device 4010 discussed herein with reference to FIG. 4) that provides outputs to the user and/or prompts the user to provide inputs (e.g., via one or more input devices, such as a keyboard, mouse, trackpad, or touchscreen, included in the other I/O devices 4012 discussed herein with reference to FIG. 4). The scientific instrument support systems disclosed herein may include any suitable GUIs for interaction with a user.

FIG. 3 depicts an example GUI 3000 that may be used in the performance of some or all of the support methods disclosed herein, in accordance with various embodiments. As noted above, the GUI 3000 may be provided on a display device (e.g., the display device 4010 discussed herein with reference to FIG. 4) of a computing device (e.g., the computing device 4000 discussed herein with reference to FIG. 4) of a scientific instrument support system (e.g., the scientific instrument support system 5000 discussed herein with reference to FIG. 5), and a user may interact with the GUI 3000 using any suitable input device (e.g., any of the input devices included in the other I/O devices 4012 discussed herein with reference to FIG. 4) and input technique (e.g., movement of a cursor, motion capture, facial recognition, gesture detection, voice recognition, actuation of buttons, etc.).

The GUI 3000 may include a data display region 3002, a data analysis region 3004, a scientific instrument control region 3006, and a settings region 3008. The particular number and arrangement of regions depicted in FIG. 3 is simply illustrative, and any number and arrangement of regions, including any desired features, may be included in a GUI 3000.

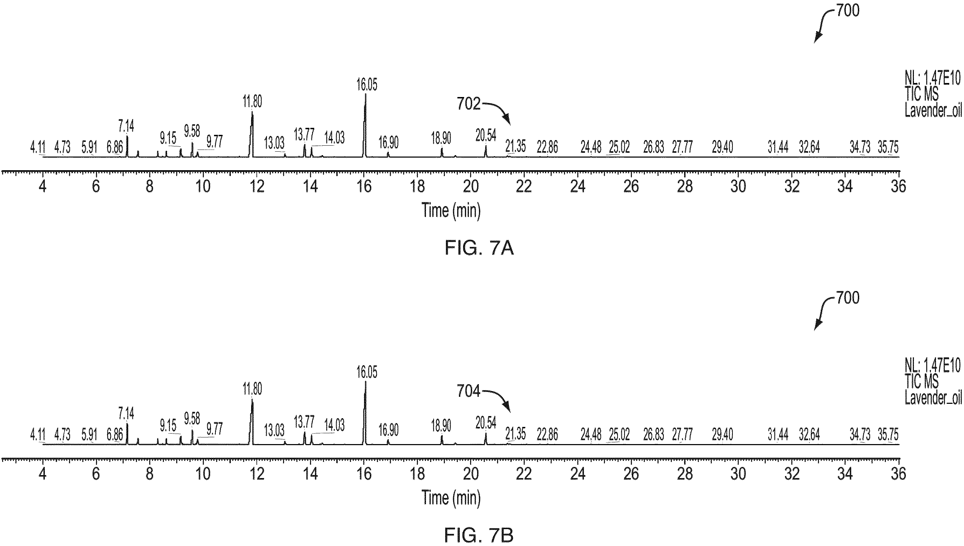

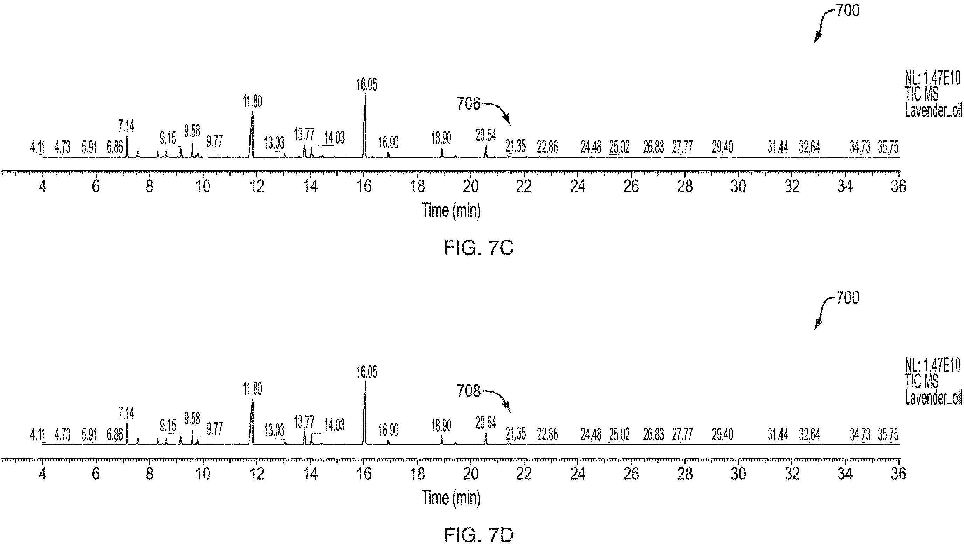

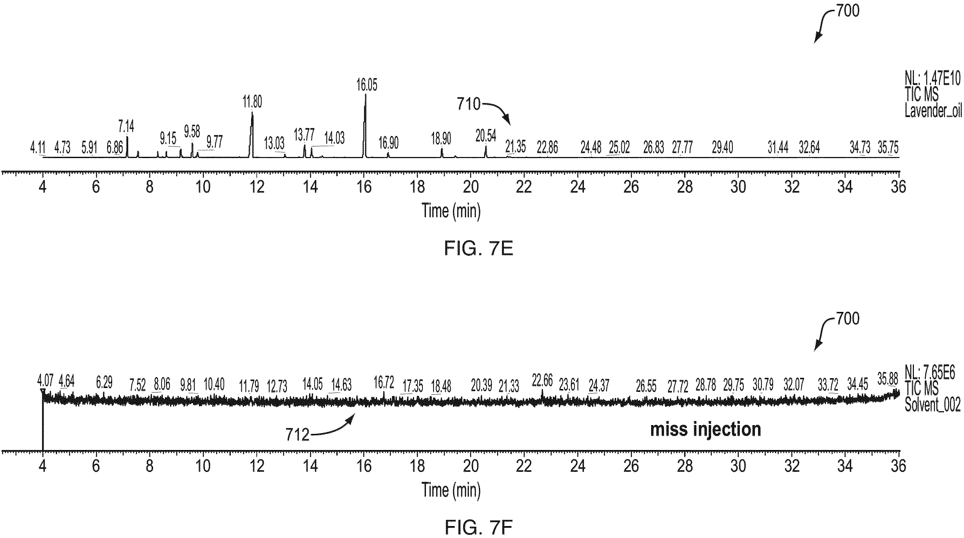

The data display region 3002 may display data generated by a scientific instrument (e.g., the scientific instrument 5010 discussed herein with reference to FIG. 5). For example, the data display region 3002 may display chromatograms obtained from various samples, such as is illustrated in FIG. 7.

The data analysis region 3004 may display the results of data analysis (e.g., the results of analyzing the data illustrated in the data display region 3002 and/or other data). For example, the data analysis region 3004 may display peaks information determined from the chromatograms, such as peak height, peak area, retention time, and the like. In some embodiments, the data display region 3002 and the data analysis region 3004 may be combined in the GUI 3000 (e.g., to include data output from a scientific instrument, and some analysis of the data, in a common graph or region).

The scientific instrument control region 3006 may include options that allow the user to control a scientific instrument (e.g., the scientific instrument 5010 discussed herein with reference to FIG. 5). For example, the scientific instrument control region 3006 may include a list of samples, methods to be performed on each sample, instrument settings like flow rates and temperatures, and the like.

The settings region 3008 may include options that allow the user to control the features and functions of the GUI 3000 (and/or other GUIs) and/or perform common computing operations with respect to the data display region 3002 and data analysis region 3004 (e.g., saving data on a storage device, such as the storage device 4004 discussed herein with reference to FIG. 4, sending data to another user, labeling data, etc.). For example, the settings region 3008 may include a setting for the threshold for identifying missed injections. The settings region 3008 may also include a section of methods for notifying the user. Additionally, the settings region may include a listing of preprogrammed responses to take in the event of an abnormal chromatogram, such as one indicative of a missed injection.

As noted above, the scientific instrument support module 1000 may be implemented by one or more computing devices. FIG. 4 is a block diagram of a computing device 4000 that may perform some or all of the scientific instrument support methods disclosed herein, in accordance with various embodiments. In some embodiments, the scientific instrument support module 1000 may be implemented by a single computing device 4000 or by multiple computing devices 4000. Further, as discussed below, a computing device 4000 (or multiple computing devices 4000) that implements the scientific instrument support module 1000 may be part of one or more of the scientific instrument 5010, the user local computing device 5020, the service local computing device 5030, or the remote computing device 5040 of FIG. 5.

The computing device 4000 of FIG. 4 is illustrated as having a number of components, but any one or more of these components may be omitted or duplicated, as suitable for the application and setting. In some embodiments, some or all of the components included in the computing device 4000 may be attached to one or more motherboards and enclosed in a housing (e.g., including plastic, metal, and/or other materials). In some embodiments, some these components may be fabricated onto a single system-on-a-chip (SoC) (e.g., an SoC may include one or more processing devices 4002 and one or more storage devices 4004). Additionally, in various embodiments, the computing device 4000 may not include one or more of the components illustrated in FIG. 4, but may include interface circuitry (not shown) for coupling to the one or more components using any suitable interface (e.g., a Universal Serial Bus (USB) interface, a High-Definition Multimedia Interface (HDMI) interface, a Controller Area Network (CAN) interface, a Serial Peripheral Interface (SPI) interface, an Ethernet interface, a wireless interface, or any other appropriate interface). For example, the computing device 4000 may not include a display device 4010, but may include display device interface circuitry (e.g., a connector and driver circuitry) to which a display device 4010 may be coupled.

The computing device 4000 may include a processing device 4002 (e.g., one or more processing devices). As used herein, the term "processing device" may refer to any device or portion of a device that processes electronic data from registers and/or memory to transform that electronic data into other electronic data that may be stored in registers and/or memory. The processing device 4002 may include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices.

The computing device 4000 may include a storage device 4004 (e.g., one or more storage devices). The storage device 4004 may include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In some embodiments, the storage device 4004 may include memory that shares a die with a processing device 4002. In such an embodiment, the memory may be used as cache memory and may include embedded dynamic random access memory (eDRAM) or spin transfer torque magnetic random access memory (STT-MRAM), for example. In some embodiments, the storage device 4004 may include non-transitory computer readable media having instructions thereon that, when executed by one or more processing devices (e.g., the processing device 4002), cause the computing device 4000 to perform any appropriate ones of or portions of the methods disclosed herein.

The computing device 4000 may include an interface device 4006 (e.g., one or more interface devices 4006). The interface device 4006 may include one or more communication chips, connectors, and/or other hardware and software to govern communications between the computing device 4000 and other computing devices. For example, the interface device 4006 may include circuitry for managing wireless communications for the transfer of data to and from the computing device 4000. The term "wireless" and its derivatives may be used to describe circuits, devices, systems, methods, techniques, communications channels, etc., that may communicate data through the use of modulated electromagnetic radiation through a nonsolid medium. The term does not imply that the associated devices do not contain any wires, although in some embodiments they might not. Circuitry included in the interface device 4006 for managing wireless communications may implement any of a number of wireless standards or protocols, including but not limited to Institute for Electrical and Electronic Engineers (IEEE) standards including Wi-Fi (IEEE 802.11 family), IEEE 802.16 standards (e.g., IEEE 802.16-2005 Amendment), Long-Term Evolution (LTE) project along with any amendments, updates, and/or revisions (e.g., advanced LTE project, ultra mobile broadband (UMB) project (also referred to as "3GPP2"), etc.). In some embodiments, circuitry included in the interface device 4006 for managing wireless communications may operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In some embodiments, circuitry included in the interface device 4006 for managing wireless communications may operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In some embodiments, circuitry included in the interface device 4006 for managing wireless communications may operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In some embodiments, the interface device 4006 may include one or more antennas (e.g., one or more antenna arrays) to receipt and/or transmission of wireless communications.

In some embodiments, the interface device 4006 may include circuitry for managing wired communications, such as electrical, optical, or any other suitable communication protocols. For example, the interface device 4006 may include circuitry to support communications in accordance with Ethernet technologies. In some embodiments, the interface device 4006 may support both wireless and wired communication, and/or may support multiple wired communication protocols and/or multiple wireless communication protocols. For example, a first set of circuitry of the interface device 4006 may be dedicated to shorter-range wireless communications such as Wi-Fi or Bluetooth, and a second set of circuitry of the interface device 4006 may be dedicated to longer-range wireless communications such as global positioning system (GPS), EDGE, GPRS, CDMA, WiMAX, LTE, EV-DO, or others. In some embodiments, a first set of circuitry of the interface device 4006 may be dedicated to wireless communications, and a second set of circuitry of the interface device 4006 may be dedicated to wired communications.

The computing device 4000 may include battery/power circuitry 4008. The battery/power circuitry 4008 may include one or more energy storage devices (e.g., batteries or capacitors) and/or circuitry for coupling components of the computing device 4000 to an energy source separate from the computing device 4000 (e.g., AC line power).

The computing device 4000 may include a display device 4010 (e.g., multiple display devices). The display device 4010 may include any visual indicators, such as a heads-up display, a computer monitor, a projector, a touchscreen display, a liquid crystal display (LCD), a light-emitting diode display, or a flat panel display.

The computing device 4000 may include other input/output (I/O) devices 4012. The other I/O devices 4012 may include one or more audio output devices (e.g., speakers, headsets, earbuds, alarms, etc.), one or more audio input devices (e.g., microphones or microphone arrays), location devices (e.g., GPS devices in communication with a satellite-based system to receive a location of the computing device 4000, as known in the art), audio codecs, video codecs, printers, sensors (e.g., thermocouples or other temperature sensors, humidity sensors, pressure sensors, vibration sensors, accelerometers, gyroscopes, etc.), image capture devices such as cameras, keyboards, cursor control devices such as a mouse, a stylus, a trackball, or a touchpad, bar code readers, Quick Response (QR) code readers, or radio frequency identification (RFID) readers, for example.

The computing device 4000 may have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultra mobile personal computer, etc.), a desktop computing device, or a server computing device or other networked computing component.

One or more computing devices implementing any of the scientific instrument support modules or methods disclosed herein may be part of a scientific instrument support system. FIG. 5 is a block diagram of an example scientific instrument support system 5000 in which some or all of the scientific instrument support methods disclosed herein may be performed, in accordance with various embodiments. The scientific instrument support modules and methods disclosed herein (e.g., the scientific instrument support module 1000 of FIG. 1 and the method 2000 of FIG. 2) may be implemented by one or more of the scientific instrument 5010, the user local computing device 5020, the service local computing device 5030, or the remote computing device 5040 of the scientific instrument support system 5000.

Any of the scientific instrument 5010, the user local computing device 5020, the service local computing device 5030, or the remote computing device 5040 may include any of the embodiments of the computing device 4000 discussed herein with reference to FIG. 4, and any of the scientific instrument 5010, the user local computing device 5020, the service local computing device 5030, or the remote computing device 5040 may take the form of any appropriate ones of the embodiments of the computing device 4000 discussed herein with reference to FIG. 4.

The scientific instrument 5010, the user local computing device 5020, the service local computing device 5030, or the remote computing device 5040 may each include a processing device 5002, a storage device 5004, and an interface device 5006. The processing device 5002 may take any suitable form, including the form of any of the processing devices 4002 discussed herein with reference to FIG. 4, and the processing devices 5002 included in different ones of the scientific instrument 5010, the user local computing device 5020, the service local computing device 5030, or the remote computing device 5040 may take the same form or different forms. The storage device 5004 may take any suitable form, including the form of any of the storage devices 5004 discussed herein with reference to FIG. 4, and the storage devices 5004 included in different ones of the scientific instrument 5010, the user local computing device 5020, the service local computing device 5030, or the remote computing device 5040 may take the same form or different forms. The interface device 5006 may take any suitable form, including the form of any of the interface devices 4006 discussed herein with reference to FIG. 4, and the interface devices 5006 included in different ones of the scientific instrument 5010, the user local computing device 5020, the service local computing device 5030, or the remote computing device 5040 may take the same form or different forms.

The scientific instrument 5010, the user local computing device 5020, the service local computing device 5030, and the remote computing device 5040 may be in communication with other elements of the scientific instrument support system 5000 via communication pathways 5008. The communication pathways 5008 may communicatively couple the interface devices 5006 of different ones of the elements of the scientific instrument support system 5000, as shown, and may be wired or wireless communication pathways (e.g., in accordance with any of the communication techniques discussed herein with reference to the interface devices 4006 of the computing device 4000 of FIG. 4). The particular scientific instrument support system 5000 depicted in FIG. 5 includes communication pathways between each pair of the scientific instrument 5010, the user local computing device 5020, the service local computing device 5030, and the remote computing device 5040, but this "fully connected" implementation is simply illustrative, and in various embodiments, various ones of the communication pathways 5008 may be absent. For example, in some embodiments, a service local computing device 5030 may not have a direct communication pathway 5008 between its interface device 5006 and the interface device 5006 of the scientific instrument 5010, but may instead communicate with the scientific instrument 5010 via the communication pathway 5008 between the service local computing device 5030 and the user local computing device 5020 and the communication pathway 5008 between the user local computing device 5020 and the scientific instrument 5010.

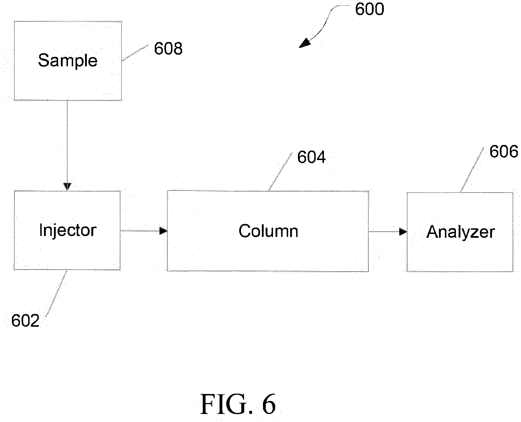

The scientific instrument 5010 may include any appropriate scientific instrument, such as a chromatography system 600 shown in FIG. 6.

The user local computing device 5020 may be a computing device (e.g., in accordance with any of the embodiments of the computing device 4000 discussed herein) that is local to a user of the scientific instrument 5010. In some embodiments, the user local computing device 5020 may also be local to the scientific instrument 5010, but this need not be the case; for example, a user local computing device 5020 that is in a user's home or office may be remote from, but in communication with, the scientific instrument 5010 so that the user may use the user local computing device 5020 to control and/or access data from the scientific instrument 5010. In some embodiments, the user local computing device 5020 may be a laptop, smartphone, or tablet device. In some embodiments the user local computing device 5020 may be a portable computing device. In some embodiments, the user local computing device 5020 may receive a notification of an abnormal chromatogram, generate an tactile, audio, or visual alert to the user, and provide the user with the ability to respond to the notification.

The service local computing device 5030 may be a computing device (e.g., in accordance with any of the embodiments of the computing device 4000 discussed herein) that is local to an entity that services the scientific instrument 5010. For example, the service local computing device 5030 may be local to a manufacturer of the scientific instrument 5010 or to a third-party service company. In some embodiments, the service local computing device 5030 may communicate with the scientific instrument 5010, the user local computing device 5020, and/or the remote computing device 5040 (e.g., via a direct communication pathway 5008 or via multiple "indirect" communication pathways 5008, as discussed above) to receive data regarding the operation of the scientific instrument 5010, the user local computing device 5020, and/or the remote computing device 5040 (e.g., the results of self-tests of the scientific instrument 5010, calibration coefficients used by the scientific instrument 5010, the measurements of sensors associated with the scientific instrument 5010, etc.). In some embodiments, the service local computing device 5030 may communicate with the scientific instrument 5010, the user local computing device 5020, and/or the remote computing device 5040 (e.g., via a direct communication pathway 5008 or via multiple "indirect" communication pathways 5008, as discussed above) to transmit data to the scientific instrument 5010, the user local computing device 5020, and/or the remote computing device 5040 (e.g., to update programmed instructions, such as firmware, in the scientific instrument 5010, to initiate the performance of test or calibration sequences in the scientific instrument 5010, to update programmed instructions, such as software, in the user local computing device 5020 or the remote computing device 5040, etc.). A user of the scientific instrument 5010 may utilize the scientific instrument 5010 or the user local computing device 5020 to communicate with the service local computing device 5030 to report a problem with the scientific instrument 5010 or the user local computing device 5020, to request a visit from a technician to improve the operation of the scientific instrument 5010, to order consumables or replacement parts associated with the scientific instrument 5010, or for other purposes.

The remote computing device 5040 may be a computing device (e.g., in accordance with any of the embodiments of the computing device 4000 discussed herein) that is remote from the scientific instrument 5010 and/or from the user local computing device 5020. In some embodiments, the remote computing device 5040 may be included in a datacenter or other large-scale server environment. In some embodiments, the remote computing device 5040 may include network-attached storage (e.g., as part of the storage device 5004). The remote computing device 5040 may store data generated by the scientific instrument 5010, perform analyses of the data generated by the scientific instrument 5010 (e.g., in accordance with programmed instructions), facilitate communication between the user local computing device 5020 and the scientific instrument 5010, and/or facilitate communication between the service local computing device 5030 and the scientific instrument 5010.

In some embodiments, one or more of the elements of the scientific instrument support system 5000 illustrated in FIG. 5 may not be present. Further, in some embodiments, multiple ones of various ones of the elements of the scientific instrument support system 5000 of FIG. 5 may be present. For example, a scientific instrument support system 5000 may include multiple user local computing devices 5020 (e.g., different user local computing devices 5020 associated with different users or in different locations). In another example, a scientific instrument support system 5000 may include multiple scientific instruments 5010, all in communication with service local computing device 5030 and/or a remote computing device 5040; in such an embodiment, the service local computing device 5030 may monitor these multiple scientific instruments 5010, and the service local computing device 5030 may cause updates or other information may be "broadcast" to multiple scientific instruments 5010 at the same time. Different ones of the scientific instruments 5010 in a scientific instrument support system 5000 may be located close to one another (e.g., in the same room) or farther from one another (e.g., on different floors of a building, in different buildings, in different cities, etc.). In some embodiments, a scientific instrument 5010 may be connected to an Internet-of-Things (IoT) stack that allows for command and control of the scientific instrument 5010 through a web-based application, a virtual or augmented reality application, a mobile application, and/or a desktop application. Any of these applications may be accessed by a user operating the user local computing device 5020 in communication with the scientific instrument 5010 by the intervening remote computing device 5040. In some embodiments, a scientific instrument 5010 may be sold by the manufacturer along with one or more associated user local computing devices 5020 as part of a local scientific instrument computing unit 5012.

In some embodiments, different ones of the scientific instruments 5010 included in a scientific instrument support system 5000 may be different types of scientific instruments 5010; for example, one scientific instrument 5010 may be a chromatographic system, such as chromatographic system 600 described in FIG. 6. In some such embodiments, the remote computing device 5040 and/or the user local computing device 5020 may combine data from different types of scientific instruments 5010 included in a scientific instrument support system 5000.

FIG. 6 illustrates a typical chromatograph system 600. In various embodiments, chromatography system 600 can be a gas chromatography system, a liquid chromatography system, an ion chromatography system, or the like. The system includes an injector 602, a column 604, and an analyzer 606. A sample 608 can be supplied to the injector 602. In various embodiments, the sample 608 can be a liquid sample. In other embodiments, the sample 608 can be a gaseous sample, such as for head space analysis. In an LC or IC system, the injector 602 can include sample loop or concentrator column into which at least a portion of the sample 608 can be loaded. In various embodiments of a GC system, the injector 602 can vaporize at least a portion of a liquid sample 608 into the gas phase. The sample 608 can be moved from the injector to the column 604 by flowing an eluent through the injector to move the sample 608 into the column 604. The column 604 includes a retention medium. In various embodiments, the retention medium can be a thin coating on the interior surface of the column 604. Alternatively, the retention medium can be in the form of beads or the like that are packed into the interior of the column 604. The retention medium can differentially retain some compounds from the sample 608 such that the amount of time necessary to transit the column is compound dependent. In this way, the compounds in the sample 608 can be separated based on the time to transit the column (retention time).

Upon exiting the column 604, the compounds can enter the analyzer 606. Various detectors can be used as part of a chromatography system including ultraviolet/visible detectors, infrared detectors, flame ionization detectors, nitrogen phosphorous detectors, electron capture detectors, thermal conductivity detectors, flame photometric detectors, mass spectrometers, and the like.

FIGs. 7A-7F illustrate a series of chromatograms. Chromatograms 702, 704, 706, 708, and 710 illustrate normal injections with chromatogram 712 showing missed injection. Rather than the peaks present in a normal chromatogram, chromatogram 712 only shows background. In a missed injection, little to no sample is injected into the column resulting in the absence of sample related peaks. In various embodiments, the missed injection can be caused by a bent syringe, a clogged needle, or misalignment of the syringe with the injection port. In the case of a bent syringe, all subsequent chromatograms will look similar to chromatogram 712 until the syringe is replaced. A clogged needle may eventually resolve itself after one or more injections and resume normal operation. In some cases, the clog may be caused by dried material in the syringe after not being used for a prolonged period of time. Often the dried material can be dissolved after one or more injection systems. In other cases, the clog may be more difficult to dislodge and repeated injection attempts may result in a bent plunger. Missed injections can also be caused by too little sample in the sample vial or the needle not being inserted far enough into the sample vial.

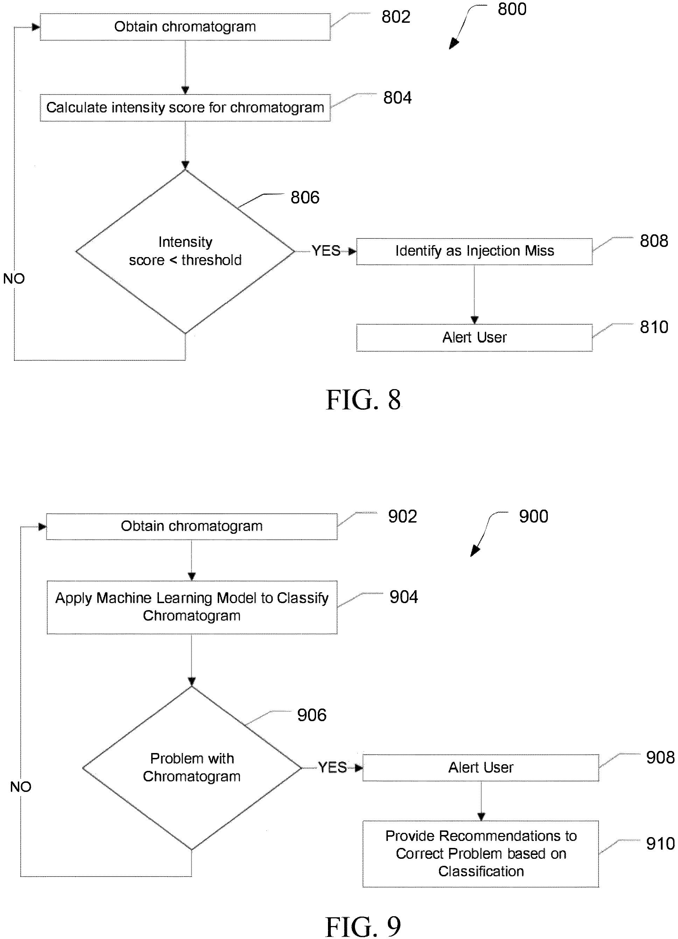

FIG. 8 is a flow diagram illustrating a method 800 of operating the chromatographic system. At 802, the system can obtain a chromatogram. For example, the scientific instrument support module can instruct an autosampler to transfer an aliquot from a sample vial, such as sample 608, to the injector, such as injector 602. The injector can supply the aliquot to the chromatography column, such as column 604. The sample can be separated on the chromatography column and the eluted compounds can be detected by a detector, such as analyzer 606. The output of the detector can be captured by the scientific instrument support module to generate a chromatogram.

At 804, an intensity score for the chromatogram can be calculated. In various embodiments, the intensity score can be a summation of the intensities across a region of the chromatogram, such was where sample peaks are expected. Alternatively, the intensity score can be an average intensity or average area of a number of largest peaks or peaks at preselected locations, such as where compounds of interest are expected. In other embodiments, the intensity score can include the number of peaks above an intensity threshold or an area above an intensity threshold. In further embodiments, a chromatographic similarity score can be calculated. The chromatographic similarity score can include factors such as number and location of peaks, peak shape, peak intensity, relative intensity of peaks, and the like. One of skill in the art would identify a number of other metrics that could be used as an intensity score for use in the methods disclosed herein.

At 806, the intensity score can be compared to a score threshold. In various embodiments, the score threshold can be determined by averaging the intensity score over as number of previous chromatograms. Depending on the metric used for the intensity score, the score threshold can be determined as a fraction of the average intensity score of the previous chromatograms. For example, the total intensity of a chromatogram from a successful injection can be several orders of magnitude higher than the total intensity of a chromatogram from a missed injection. As such, the threshold may be set at not greater than 1/10 the average intensity score, such as not greater than 1/100 the average intensity score. For other metrics, such as a number of peaks above a threshold, the difference between a successful injection and a missed injection may be smaller, such as not greater than 75%, even not greater than 50%. Alternatively, the threshold can be a user selectable parameter without the need for the scientific instrument support module to analyze chromatograms from prior successful injections.

If the intensity score is not below the threshold, the system can return to 802 to obtain another chromatogram. Alternatively, if the intensity score is below the threshold, the scientific instrument support module can identify the chromatogram as an injection miss at 808 and can notify the user at 810. In various embodiments, the user can be notified by an audio or visual alert from the instrument or an electronic message sent to the user, such as an email, SMS message, alert sent to a mobile device, or the like. In some embodiments, the scientific instrument support module can be configured to pause or stop the chromatographic system from processing further samples until a user action is performed. In various embodiments, a user can set a threshold to trigger an alert and a separate threshold to pause or stop the system. For example, a user might set the alert trigger at closer to the expected value and the stop/pause trigger at values further from the expected value.

FIG. 9 is a flow diagram illustrating a method 800 of operating the chromatographic system. At 802, the system can obtain a chromatogram, such as previously described in relation to FIG. 8.

At 904, a machine learning model can be used to classify the chromatogram. In various embodiments, the machine learning model can be trained on a large number of chromatograms that include successful injections and missed injections of various types. Additionally, the training set can include chromatograms with other errors, such as leaks. The machine learning model can be trained classify the chromatogram into normal chromatograms or various classes of chromatographic errors including missed injections. In various embodiments, the machine learning model can identify chromatographic errors such as significant shifts in retention times and poor peak shape.

In various embodiments, the machine learning model can be applied by the processing device of the scientific instrument, the processing device of the user local computing device, the processing device of the service local computing device, or the processing device of the remote computing device. In various embodiments, the machine learning model can be applied using a single computing device or may be applied in a distributed fashion, such as a cloud implementation.

At 906, the scientific instrument support module can determine if there is a problem with the chromatogram based on the classification from the machine learning model. If the machine learning model classifies the chromatogram as normal or successful, the system can return to 902 to obtain another chromatogram. Alternatively, when the chromatogram is classified as one of the classes of chromatographic errors, the system can alert the user at 908. Additionally, at 910, the system can provide recommendations to the user on how to correct the problem based on the classification by the machine learning model. For example, when classified as a missed injection, the system can recommend the user to check for a bent, broken, or clogged needle or to check the volume in the sample vial. Alternatively, when the chromatogram is classified as representing a leak, the system can recommend the user check various values and fittings for the leak. In various embodiments, the user can be notified by an audio or visual alert from the instrument or an electronic message sent to the user, such as an email, SMS message, alert sent to a mobile device, or the like. In some embodiments, the scientific instrument support module can be configured to pause or stop the chromatographic system from processing further samples until a user action is performed.

While the present teachings are described in conjunction with various embodiments, it is not intended that the present teachings be limited to such embodiments. On the contrary, the present teachings encompass various alternatives, modifications, and equivalents, as will be appreciated by those of skill in the art.

Further, in describing various embodiments, the specification may have presented a method and/or process as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims. In addition, the claims directed to the method and/or process should not be limited to the performance of their steps in the order written, and one skilled in the art can readily appreciate that the sequences may be varied and still remain within the scope of the various embodiments.

## Claims

1. A method for scientific instrument support, comprising:
obtaining a chromatographic data;
calculating an intensity score for the chromatographic data;
identifying an injection miss when the intensity score is below a score
threshold; and
notifying a user of an injection miss.

2. The method of claim 1, wherein the intensity score includes a total signal intensity, an average signal intensity, average area of a number of peaks, a number of peaks above an intensity threshold, an area above the intensity threshold, or a chromatographic similarity score.

3. The method of claim 1 or claim 2, further comprising pausing or stopping a chromatography system until the cause of the injection miss is corrected or a user input to resume or restart is received.

4. One or more non-transitory computer readable media having instructions thereon that, when executed by one or more processing devices of a scientific instrument support apparatus, cause the scientific instrument support apparatus to perform the method of any one of claims 1-3.

5. A scientific instrument support apparatus comprising
first logic to obtain a chromatographic data;
second logic to:
calculate an intensity score for the chromatographic data; and
identify an injection miss when the intensity score is below a threshold;
and
third logic to notify a user of an injection miss.

6. The scientific instrument support system of claim 5, wherein the intensity score includes a total signal intensity, an average signal intensity, average area of a number of peaks, a number of peaks above an intensity threshold, an area above the intensity threshold, or a chromatographic similarity score.

7. The scientific instrument support system of claim 5 or claim 6, wherein the third logic is further configured to pause or stop a chromatography system until the cause of the injection miss is corrected or a user input to resume or restart is received.

8. The scientific instrument support system of claim 5, claim 6 or claim 7,
wherein the first logic, the second logic, and the third logic are implemented by a common computing device.

9. The scientific instrument support system of any of claims 5-7, wherein at least one of the first logic, the second logic, and the third logic are implemented by a computing device remote from the scientific instrument.

10. The scientific instrument support system of any of claims 5-7, wherein at least one of the first logic, the second logic, and the third logic are implemented by a user computing device.

11. The scientific instrument support system of any of claims 5-7, wherein at least one of the first logic, the second logic, and the third logic are implemented in the scientific instrument.
